# NEUE EUROPÄISCHE PATENTSCHRIFT

(11) **EP 2 324 832 B2**
(45) Veröffentlichungstag und Bekanntmachung des Hinweises auf die Entscheidung über den Einspruch: **30.11.2016**
(45) Hinweis auf die Patenterteilung: 14.05.2014
(21) Anmeldenummer: 09014013.8
(22) Anmeldetag: 09.11.2009
(51) Int. Cl.: A61K 45/06, A61K 31/4415, A61K 31/375, A61K 31/519, A61K 31/525, A61K 31/714

(54) **Nahrungsergänzungsmittel bei Einnahme von hormonellen Kontrazeptiva**
Nutritional supplement while taking hormonal contraceptives
Complément alimentaire lors d'une prise de contraceptifs hormonaux

(43) Veröffentlichungstag der Anmeldung: 25.05.2011
(73) Patentinhaber: Biogena Naturprodukte GmbH & Co KG, 5020 Salzburg (AT)
(72) Erfinder: Schmidbauer, Albert, 5020 Salzburg (AT); Leisser, Christina, Dr., 1070 Wien (AT)
(74) Vertreter: Graf von Stosch, Andreas

(56) Entgegenhaltungen:
- WO-A1-98/04269
- DE-U1-202005 003 148
- DE-U1-202005 020 102
- US-A1- 2005 164 977
- US-B1- 7 136 820
- "Multibionta plus Mineral" In: "Rote Liste 2007" 1. Januar 2007 (2007-01-01), Verlag Rote Liste Service GmbH , Frankfurt/Main , XP002575615 * Absatz 84 133 *
- DATABASE EMBASE [Online] ELSEVIER SCIENCE PUBLISHERS, AMSTERDAM, NL; 1972, SPELLACY W N ET AL: "The effects of vitamin b, on carbohydrate metabolism in women taking steroid contraceptives: preliminary report" XP002575616 Database accession no. EMB-0008690005
- DATABASE MEDLINE [Online] US NATIONAL LIBRARY OF MEDICINE (NLM), BETHESDA, MD, US; 1974, BRIGGS M ET AL: "Changes in biochemical indices of vitamin nutrition in women using oral contraceptives during treatment with "Surbex 500"." XP002575617 Database accession no. NLM4281739

## Beschreibung

Die vorliegende Erfindung betrifft Vitamin B6-haltige Zusammensetzungen, insbesondere Zusammensetzungen die Vitamin B6, Vitamin B2, Vitamin B12, Folsäure, Vitamin C, Zink und Magnesium enthalten. Ferner betrifft die vorliegende Erfindung die Verwendung dieser Zusammensetzungen als Nahrungsergänzungsmittel, insbesondere zur Nahrungsergänzung bei Personen, die weibliche Hormonpräparate zu sich nehmen, z.B. hormonelle Kontrazeptiva, und/oder zur Nahrungsergänzung insbesondere bei der Einnahme derselben oder auch nach Absetzung der Einnahme dieser Präparate. Ferner betrifft die vorliegende Erfindung die Verwendung dieser Zusammensetzungen zur Prävention und Behandlung von Ernährungsmangelzuständen, insbesondere infolge der Einnahme von weiblichen Hormonpräparaten.

Hormonelle Kontrazeptiva sind Hormonpräparate bestehend aus unterschiedlichen Kombinationen von Östrogenen und Gestagenen, mit Ausnahme der Minipille, die lediglich ein Gestagen enthält. Die gängigste Form dieser Kontrazeptiva sind die oralen Kontrazeptiva, landläufig als "die Pille" bezeichnet. Hormonelle Kontrazeptiva können das Risiko eines Auftretens von thromboembolischen Erkrankung wie Lungenembolie, Herzinfarkt und Schlaganfall erhöhen. Weitere mögliche unerwünschte Wirkungen sind Gewichtszunahme, Ödeme, Übelkeit, Erbrechen und Blutdruckanstieg. Darüber hinaus führt die Einnahme von (oralen) Kontrazeptiva zu umfassenden Veränderungen im Stoffwechsel, u. a. auch mit großem Einfluss auf den Vitaminhaushalt. Medizinisch besonders bedeutsam sind dabei Absenkungen des Vitamin B6-Spiegels, der durch den beschleunigten Umsatz dieses Vitamins unter Hormoneinwirkung verursacht wird. Daneben können hormonelle Kontrazeptiva zu erniedrigten Spiegeln anderer Vitamine führen, insbesondere von Vitaminen der B-Gruppe und von Vitamin C. Die Folsäure, auch Vitamin B9 genannt, ist dabei von besonderer Bedeutung. Eine Anzahl von Erkrankungen wird als mit einem Folatmangel in Zusammenhang stehend angesehen. So kann die Verabreichung von Folaten zum Beispiel in Form von Folsäure das Risiko von Herz-/Kreislauferkrankungen sowie bestimmten malignen Erkrankungen (wie zum Beispiel Brust- und Colonkarzinom) minimieren. Nach Absetzen der Pille und Konzeption können niedrige Folsäurespiegel fatale Auswirkungen auf die Entwicklung des Ungeborenen haben. So haben Frauen mit niedrigen Folatspiegeln gegenüber solchen mit ausreichend hohen Folatspiegeln ein erhöhtes Risiko, Kinder zu gebären, die an angeborenen Fehlbildungen wie Neuralrohr-, Ventrikelklappen- und Urogenitaldefekten leiden. Neuralrohrdefekte sind die häufigsten angeborenen Fehlbildungen des Zentralnervensystems. Sie entstehen durch einen unvollständigen Verschluss des Neuralrohrs etwa in der dritten bis vierten Woche der embryonalen Entwicklung. Zu den Neuralrohrdefekten gehören die Spina bifida (teilweise mit Meningozele oder Meningomyelozele), die Enzephalozele bzw. Anenzephalien, die durch das teilweise oder vollständige Fehlen von Hirnarealen gekennzeichnet sind. Kinder mit Anenzephalie sind praktisch nicht überlebensfähig.

Der vorliegenden Erfindung lag daher die Aufgabe zugrunde, Mittel bereitzustellen, die den mit der Einnahme von hormonellen Kontrazeptiva einhergehenden Störungen im Metabolismus, insbesondere im Vitamin- und Spurenelementhaushalt, entgegenwirken.

Dies wird durch den in den Ansprüchen beschriebenen Gegenstand erreicht.

In einem ersten Aspekt betrifft die vorliegende Erfindung Zusammensetzungen, die Vitamin B6 oder Salze davon umfassen. Insbesondere betrifft die vorliegende Erfindung Zusammensetzungen, die neben Vitamin B6 (oder Salze davon) mindestens einen weitere Vitamin und/oder mindestens ein Spurenelement (oder jeweils Salze davon) umfassen.

Die erfindungsgemäßen Zusammensetzungen umfassen Vitamin B2, Folsäure, Vitamin B12 und Vitamin C.

Die erfindungsgemäßen Zusammensetzungen die zwei Spurenelemente Magnesium und Zink.

Die Zusammensetzungen gemäß der Erfindung umfassen daher Vitamin B2, Vitamin B6, Vitamin B12, Folsäure, Vitamin C, Zink und Magnesium (und/oder jeweils Salze davon).

Ferner können die gemäß der Erfindung zu verwendenden Zusammensetzungen beispielsweise ein, zwei oder drei weitere Substanzen ausgewählt aus der Gruppe bestehend aus Eisen, (Bio)Flavonoide und Piperin umfassen.

Ebenso können die gemäß der Erfindung zu verwendenden Zusammensetzungen Vitamin B2, Vitamin B6, Vitamin B12, Folsäure, Vitamin C, Zink, Magnesium, und ein, zwei oder drei weitere Substanzen ausgewählt aus der Gruppe bestehend aus Eisen, (Bio)Flavonoiden und Piperin umfassen.

In einer bestimmten Ausführungsform können die gemäß der Erfindung zu verwendenden Zusammensetzungen daher Vitamin B2, Vitamin B6, Vitamin B12, Folsäure, Vitamin C, Zink, Magnesium, Eisen, (Bio)Flavonoide und Piperin umfassen.

Ferner können die erfindungsgemäßen Zusammensetzungen im Wesentlichen (oder vollständig) frei sein von bestimmten anderen Substanzen, die im Stand der Technik zum Teil für Nahrungsergänzungsmittel vorgeschlagen werden.

In einer besonderen Ausführungsform bedeutet "im Wesentlichen frei", dass die entsprechenden Substanzen weniger als etwa 5%, vorzugsweise weniger als etwa 1%, vorzugsweise weniger als etwa 0,5%, noch bevorzugter weniger als etwa 0,25%, noch bevorzugter weniger als etwa 0,1 % noch bevorzugter weniger als etwa 0,05%, noch bevorzugter weniger als etwa 0,01%, noch bevorzugter weniger als etwa 0,001%, noch bevorzugter weniger als etwa 0,0001%, noch bevorzugter weniger als etwa 0,00001 %, am bevorzugtesten etwa 0% der Feststoffe der Zusammensetzung ausmachen (d.h. ohne Berücksichtigung von Lösungsmitteln wie Wasser etc.). "Im Wesentlichen frei" bedeutet insbesondere, dass die entsprechende Substanz nicht separat zugesetzt wurde. So kann im Wesentlichen frei von Cobalt bedeuten, dass eine Zusammensetzung, die z.B. Cobalamine (Vitamin B12) enthält, zwar von Cobalaminen komplexiertes Cobalt umfasst, jedoch kein anderweitig zugesetztes Cobalt. Analoges gilt z.B. auch für Extrakte, z.B. Pflanzenextrakte, d.h. eine Zusammensetzungen die gemäß der Erfindung einen Pflanzenextrakt enthält und im Wesentlichen frei von z.B. Proteinen ist, kann zwar die im Extrakt ggf. enthaltenen Proteine umfassen, jedoch keine anderweitig zugesetzten Proteine.

Die Zusammensetzungen der vorliegenden Erfindung sind vollständig frei von Vitamin A. Die Zusammensetzungen der vorliegenden Erfindung können im Wesentlichen (oder vollständig) frei sein von ein, zwei, drei, vier, fünf, sechs oder sieben der Vitamine (und/oder deren Salzen), die ausgewählt werden aus der Gruppe bestehend aus Vitamin B1, Vitamin B3, Vitamin B5, Vitamin B7, Vitamin D, Vitamin E, Vitamin K und deren Salzen. Insbesondere können die Zusammensetzungen der vorliegenden Erfindung im Wesentlichen (oder vollständig) frei sein von Vitamin B7 und/oder Vitamin K.

Ferner können die Zusammensetzungen der vorliegenden Erfindung im Wesentlichen (oder vollständig) frei sein von (z.B. isolierten und/oder synthetischen): Aminosäuren (und/oder deren Salzen), Peptiden, und/oder Proteinen. Insbesondere können die Zusammensetzungen der vorliegenden Erfindung frei sein von Alanin, Arginin, Asparagin, Asparaginsäure, Cystein, Cystin, Glutamin, Glutaminsäure, Glycin, Histidin, Isoleucin, Leucin, Lysin, Methionin, Phenylalanin, Prolin, Serin, Threonin, Tryptophan, Tyrosin und/oder Valin, und/oder den entsprechenden Salzen wie z.B. Glutamat und Aspartat.

Ferner können die Zusammensetzungen der vorliegenden Erfindung im Wesentlichen (oder vollständig) frei sein von (z.B. isolierten und/oder synthetischen) Hormonen. Insbesondere können die Zusammensetzungen der vorliegenden Erfindung im Wesentlichen (oder vollständig) frei sein von (z.B. isolierten und/oder synthetischen): weiblichen Hormonen wie Gestagenen, Östrogenen, Follikel-stimulierendem Hormon, Luteinisierendem Hormon und/oder Choriongonadotropin. Insbesondere können die Zusammensetzungen der vorliegenden Erfindung im Wesentlichen (oder vollständig) frei sein von (z.B. isolierten und/oder synthetischen) Gestagenen und/oder Östrogenen.

Ferner können die Zusammensetzungen der vorliegenden Erfindung im Wesentlichen (oder vollständig) frei sein von (z.B. isolierten und/oder synthetischen): Nukleinsäuren, DNA, RNA, Kohlehydraten, Fetten, Lipiden und/oder Fettsäuren (insbesondere Omega-3-Fettsäuren).

Ferner können die Zusammensetzungen der vorliegenden Erfindung im Wesentlichen (oder vollständig) frei sein von (z.B. isolierten): weiteren Spurenelementen, insbesondere von Chrom, Cobalt, Iod, Kupfer, Mangan, Molybdän, Selen, Arsen, Fluor, Nickel, Silizium, Vanadium, Zinn, Bor und/oder Rubidium. In den Fällen, in denen die in der vorliegenden Erfindung genannten Zusammensetzungen in bestimmten Ausführungsformen explizit das Vorliegen bestimmter Spurenelemente wie Magnesium, Zink oder Eisen verlangen, bedeutet im Wesentlichen (oder vollständig) frei von weiteren Spurenelementen, dass die Zusammensetzung abgesehen von den unmittelbar spezifizierten Spurenelementen im Wesentlichen (oder vollständig) frei von anderen (weiteren) Spurenelementen ist.

Ferner können die Zusammensetzungen der vorliegenden Erfindung im Wesentlichen (oder vollständig) frei sein von (z.B. isoliertem oder synthetischem) Coenzym Q10 und/oder dessen Salzen.

Ferner können die Zusammensetzungen der vorliegenden Erfindung ggf. im Wesentlichen (oder vollständig) frei sein von: (weiteren) natürlichen Extrakten, insbesondere pflanzlichen Extrakten.

In den Fällen, in denen die in der vorliegenden Erfindung genannten Zusammensetzungen in bestimmten Ausführungsformen explizit das Vorliegen bestimmter Extrakte verlangen, bedeutet im Wesentlichen (oder vollständig) frei von weiteren natürlichen Extrakten, insbesondere pflanzlichen Extrakten, dass die Zusammensetzung abgesehen von den unmittelbar spezifizierten Extrakten im Wesentlichen (oder vollständig) frei von anderen (weiteren) natürlichen Extrakten, insbesondere anderen pflanzlichen Extrakten ist. Insbesondere können die erfindungsgemäßen Zusammensetzungen im Wesentlichen (oder vollstände frei von sein von Bockshornkleesamen-Extrakt, Ginko biloba-Extrakt, Allium sativum-Extrakt, Hypericum perforatum-Extrakt, Panax ginseng-Extrakt, Vitis vinifera-Extrakt, Guarana-Extrakt, Cimicifuga racemosa-Extrakt" Turnera aphrodisiaca-Extrakt, Grünteeextrakt, Garcinia cambogia-Extrakt und/oder Aloe vera-Extrakt. Ferner können die erfindungsgemäßen Zusammensetzungen im Wesentlichen (oder vollständig) frei sein von Wirkstoffen, die beispielsweise durch Extraktion aus pflanzlichem Material isoliert (oder chemisch synthetisiert) werden können.

Es versteht sich, dass der hier verwendete Begriff Zusammensetzung weder ein natürliches Lebensmittel als solches darstellt noch eine Kombination natürlicher Lebensmittel. Vielmehr liegen die Komponenten der Zusammensetzung insbesondere in konzentrierter Form vor.

Die Komponenten der erfindungsgemäßen Zusammensetzungen können als solche und/oder auch jeweils als Salze vorliegen. Besonders bevorzugt sind pharmazeutisch verträgliche Salze, die sowohl pharmakologisch wie auch pharmazeutisch verträglich sind. Solche pharmakologisch und pharmazeutisch verträgliche Salze können Alkali- oder Erdalkalimetall-Salze sein, vorzugsweise Natrium-, Kalium-, Magnesium oder Calcium-Salze. Besonders bevorzugt ist das Calciumsalz. Es versteht sich, dass die Komponenten der erfindungsgemäßen Zusammensetzung auch jeweils in unterschiedlicher (kombinierter) Form vorliegen können, z.B. zwei oder mehrer Formen von Vitamin B12 nebeneinander (z.B. Hydroxycobalmin und Cyanocobalmin).

Vitamin B6, auch Pyridoxin genannt, ist ein zentrales Element der Zusammensetzungen der vorliegenden Erfindung. Der Begriff stellt einen Sammelbegriff für verschiedene Derivate von 4,5-Bis(hydroxymethyl)-2-methylpyridin-3-ol dar. Sie unterscheiden sich durch unterschiedliche Substituenten in Position 4, die an der Coenzymfunktion beteiligt ist. Phosphorylierte Vitamin-B6-Derivate wirken als Coenzyme in zahlreichen enzymatischen Reaktionen, vorwiegend im Aminosäurestoffwechsel. Eine weitere wichtige Aufgabe übernimmt das Pyridoxalphosphat (PLP oder PALP, ein Pyridoxin-Derivat) als Cofaktor bei der Synthese der δ-Aminolävulinsäure, eines Zwischenproduktes in der endogenen Häm-Synthese. Der Begriff Vitamin B6, wie hierin verwendet, umfasst alle für den (menschlichen) Körper zugänglichen und verwertbaren Vitamin B6-Varianten und -salze. In den erfindungsgemäßen Zusammensetzungen kann Vitamin B6 vorzugsweise als Pyridoxinhydrochlorid und/oder Pyridoxal-5-phosphat vorliegen.

Vitamin B2 (auch Riboflavin genannt), ist für höhere Tiere, und damit auch für den Menschen, eine essentielle Verbindung. Vitamin B2 findet in Nahrungsergänzungsmitteln weit verbreitet Anwendung. Darüber hinaus kann Vitamin B2 als Lebensmittelfarbe eingesetzt werden. Biologisch aktives Riboflavin ist z.B. Flavinmononukleotide (FMN) or Flavinadenindinukleotide (FAD). Diese aktiven Formen und ihrer reduzierten Gegenstücke, FMNH2 und FADH2, können beispielsweise im Rahmen der vorliegenden Erfindung verwendet werden. Der Begriff Vitamin B2, wie hierin verwendet, umfasst alle für den (menschlichen) Körper zugänglichen und verwertbaren Vitamin B2-Varianten, -quellen und -salze. Vitamin B2 kann vorzugsweise in den erfindungsgemäßen Zusammensetzungen als Riboflavin-5-phosphat und/oder als Natriumriboflavin vorliegen.

Cobalamine (Vitamin B12) bilden eine biochemische Stoffgruppe, der gemeinsam ist, dass alle Vertreter das Spurenelement Cobalt enthalten. Ihr wichtigster Vertreter ist Adenosylcobalamin, genauer ausgedrückt 5'-Desoxyadenosylcobalamin. Dabei handelt es sich um ein wasserlösliches Vitamin, das die unmittelbar aktive Form des Vitamins B12 repräsentiert. Des Weiteren gehören zur Vitamin-B12-Gruppe die Speicherformen Aquocobalamin (Aquacobalamin, B12a) und Hydroxocobalamin (Vitamin B12b), des weiteren Nitritocobalamin (Vitamin B12c) und die eigentlichen biologisch wirksamen Formen, die Co-Enzyme Methylcobalamin (Methyl B12) und Adenosylcobalamin. Daneben gibt es noch Cyanocobalamin, das etwas stabiler ist. Der Begriff Vitamin B12, wie hierin verwendet, umfasst alle für den (menschlichen) Körper zugänglichen und verwertbaren Vitamin B12-Varianten, -quellen und -salze. In den erfindungsgemäßen Zusammensetzungen wird Vitamin B12 vorzugsweise als Hydroxycobalamin, Cyanocobalamin, Methycobalamin, und/oder Adenosylcobalamin verwendet.

Die Folsäure, auch Vitamin B9, Vitamin B11 (ungebräuchlich) oder Folat genannt, ist die Vorstufe des Coenzyms Tetrahydrofolsäure (THF). THF wirkt besonders als Lieferant von Methyl- (CH3-), Methenyl- (CH2=) und Formyl-Gruppen (HCO-) mit an der Synthese von Purinbasen und von desoxy-Thymidinmonophosphat (dTMP). Außerdem wirkt THF als Coenzym bei der Methylierung von Homocystein zu Methionin. Aufgrund der Beteiligung an der Synthese von DNA spielt die Folsäure daher insbesondere in der Schwangerschaft, sowie bei sich häufig teilenden Zellen (z. B. Knochenmark) eine entscheidende Rolle. Der Begriff Folsäure, wie hierin verwendet, umfasst alle für den (menschlichen) Körper zugänglichen und verwertbaren Folsäure-Varianten, -quellen und -salze. Vorzugsweise wird für die erfindungsgemäßen Zusammensetzungen Folsäure als Pteroylmonoglutaminsäure, Pteroylmonoglutamat, Pteroylpolyglutamat und/oder Calcium-L-Methylfolat verwendet.

Vitamin C, auch Ascorbinsäure genannt, wird im Körper für zahlreiche Zwecke benötigt. Die bedeutendste Funktion ist seine Wirkung als Reduktionsmittel. Der Begriff Vitamin C, wie hierin verwendet, umfasst alle für den (menschlichen) Körper zugänglichen und verwertbaren Vitamin C-Varianten, -quellen und -salze. In den erfindungsgemäßen Zusammensetzungen kann Vitamin C insbesondere in Form von L-Ascorbinsäure, Natrium-L-Ascorbat, Kalium-L-Ascorbat, Calcium-L-Ascorbat, und/oder L-Ascorbyl-6-palmitat vorliegen.

Zink ist ein essentielles Spurenelement für den Stoffwechsel. Es kann nicht gespeichert werden und muss dem Körper ständig neu zugeführt werden. Es ist Bestandteil einer Vielzahl von Enzymen, beispielsweise der RNA-Polymerase. Zink hat zentrale Bedeutung im Zucker-, Fett- und Eiweißstoffwechsel, bei der DNA-Synthese sowie beim Zellwachstum. Auch etliche Hormone benötigen Zink für ihre Funktion. Der Begriff Zink, wie hierin verwendet, umfasst alle für den (menschlichen) Körper zugänglichen und verwertbaren Zinkquellen und -salze. Für die erfindungsgemäßen Zusammensetzungen kann Zink vorzugsweise als Zinkacetat, Zinkchlorid, Zinkcitrat, Zinkglukonat, Zinklactat, Zinkoxid, Zinkcarbonat, Zinksulfat, Zinkaspertat, Zink-L-aspartat, Zink-Aminosäurechelat, Zinkglycinat, Zinklysinat, Zinkmalat, Zink-L-monomethionin, und/oder Zinkpidolat vorliegen.

Magnesium ist ebenfalls ein essentielles Spurenelement. Magnesium muss dem Körper täglich in ausreichender Menge zugeführt werden. Magnesium ist an zahlreichen Enzymreaktionen als Enzymbestandteil oder Coenzym beteiligt, zudem beeinflussen freie Mg-Ionen das Potential an den Zellmembranen. Der Begriff Magnesium, wie hierin verwendei, umfasst alle für den (menschlichen) Körper zugänglichen und verwertbaren Magnesiumquellen und -salze. Magnesium kann in den erfindungsgemäßen Zusammensetzungen vorzugsweise als Magnesiumacetat, Magnesiumcarbonat, Magnesiumchlorid, Magnesiumcitrat, Magnesiumglukonat, Magnesiumglycerophosphat, Magnesiumorthophosphat, Magnesiumlactat, Magnesiumhydroxid, Magnesiumsulfat, Magnesium-Acetyl-Taurinat, Magnesiumtaurat, Magnesium-Aminosäurechelat, Magnesiumglycinat, Magnesium-L-Lysinat, Magnesiummalat, Magnesiumpidolat, Magnesiumorotat und/oder Magnesium-Kaliumcitrat vorliegen.

Eisen ist ebenfalls ein essentielles Spurenelement. Es ist bei Tieren und somit auch beim Menschen vor allem für die Blutbildung notwendig. Als Zentralatom des Kofaktors Häm b in Hämoglobin und Myoglobin und in Cytochromen ist es bei vielen Tieren und beim Menschen für Sauerstoff transport und -speicherung sowie für die Elektronenübertragung verantwortlich. Ferner ist Eisen wichtiger Bestandteil vieler Enzyme, beispielsweise von Nitrogenasen, Hydrogenasen, Katalase, etc. Der Begriff Eisen, wie hierin verwendet, umfasst alle für den (menschlichen) Körper zugänglichen und verwertbaren Eisenquellen und -salze. Eisen kann - sofern es in den erfindungsgemäßen Zusammensetzungen Verwendung findet - vorzugsweise als 2-wertige Eisenverbindung vorliegen, insbesondere als Eisencarbonat, Eisencitrat, Eisenglukonat, Eisenfumarat, Eisenlactat, Eisensulfat, Eisenbisglycinat, Eisenphosphat, Eisenpidolat, und/oder Eisen-II-Taurat.

Flavonoide sind eine Gruppe von wasserlöslichen Pflanzenfarbstoffen und spielen eine wichtige Rolle im Stoffwechsel vieler Pflanzen. Sie gehören zu den Polyphenolen. Die meisten Flavonoide sind an Glukose oder Rhamnose gebunden - daher nennt man sie Glykoside. Flavonoide haben diverse vorteilhafte Auswirkungen auf die Gesundheit. Der Begriff (Bio-)Flavanoide, wie hierin verwendet, umfasst alle für den (menschlichen) Körper zugänglichen und verwertbaren Flavanoidquellen. (Bio-)Flavanoide können - sofern sie in den erfindungsgemäßen Zusammensetzungen Verwendung finden - in den erfindungsgemäßen Zusammensetzungen vorzugsweise als pflanzlicher Extrakt vorliegen, insbesondere als Citrusextrakt und/oder Holunderextrakt.

Piperin ist ein Alkaloid und das Hauptalkaloid des schwarzen Pfeffers. Piperin regt den Stoffwechsel sowie die Sekretion an. Daneben wirkt es antimikrobiell. Der Begriff Piperin, wie hierin verwendet, umfasst alle für den (menschlichen) Körper zugänglichen und verwertbaren Piperinquellen, synthetisch wie natürlich. Piperin kann - sofern es in den erfindungsgemäßen Zusammensetzungen Verwendung findet - in den erfindungsgemäßen Zusammensetzungen vorzugsweise als pflanzlicher Extrakt vorliegen, insbesondere als Schwarzer Pfeffer-Extrakt.

Vorzugsweise liegen die mgl. Komponenten der erfindungsgemäßen Zusammensetzungen (wie oben angeführt) jeweils in einer (Tages-)Dosierung vor, die etwa 15% bis etwa 1000% der empfohlenen Tagesdosis entsprechen (z.B. bezogen auf Richtlinie der EU 2008/100/EG). Selbstverständlich können dabei die einzelnen Komponenten in unterschiedlichen Prozentmengen vorliegen.

Vorzugsweise liegt die Komponente Vitamin B6 in den erfindungsgemäßen Zusammensetzungen in einer (Tages-)Dosierung von etwa 50 bis etwa 510% der empfohlenen Tagesdosis, insbesondere von etwa 100% bis etwa 500% der empfohlenen Tagesdosis vor. Beispielsweise kann die Vitamin B6-Komponente (d.h. tatsächlicher B6 Anteil ohne Berücksichtigung z.B. des Gewichts eines etwaigen Gegenions etc.) in einer Menge von etwa 0,7 mg bis etwa 7,7 mg, von etwa 0,84 mg bis etwa 7,56 mg, von etwa 0,98 mg bis etwa 7,42 mg, von etwa 1,12 mg bis etwa 7,28 mg, von etwa 1,26 mg bis etwa 7,14 mg, von etwa 1,4 mg bis etwa 7 mg, von etwa 1,54 mg bis etwa 6,86 mg, von etwa 1,68 mg bis etwa 6,72 mg, von etwa 1,82 mg bis etwa 6,58 mg, von etwa 1,96 mg bis etwa 6,44 mg, von etwa 2,1 mg bis etwa 6,3 mg, von etwa 2,24 mg bis etwa 6,16 mg, von etwa 2,38 mg bis etwa 6,02 mg, von etwa 2,52 mg bis etwa 5,88 mg, von etwa 2,66 mg bis etwa 5,74 mg, von etwa 2,8 mg bis etwa 5,6 mg, von etwa 2,94 mg bis etwa 5,46 mg, von etwa 3,08 mg bis etwa 5,32 mg, von etwa 3,22 mg bis etwa 5,18 mg, von etwa 3,36 mg bis etwa 5,04 mg, von etwa 3,5 mg bis etwa 4,9 mg, von etwa 3,64 mg bis etwa 4,76 mg, von etwa 3,78 mg bis etwa 4,62 mg, von etwa 3,92 mg bis etwa 4,48 mg, von etwa 4,06 mg bis etwa 4,34 mg in den erfindungsgemäßen Zusammensetzungen vorliegen oder in jeglicher Kombination dieser Bereiche (z.B. von etwa 0,7 mg bis etwa 5,88 mg etc.). Besonders bevorzugt als Tagesdosis ist eine Menge von etwa 6 mg Vitamin B6 in den erfindungsgemäßen Zusammensetzungen.

Vorzugsweise liegt die Komponente Vitamin B2 in den erfindungsgemäßen Zusammensetzungen in einer (Tages-)Dosierung von etwa 20% bis etwa 550% der empfohlenen Tagesdosis, insbesondere von etwa 100% bis etwa 500% der empfohlenen Tagesdosis vor. Beispielsweise kann die Vitamin B2-Komponente (d.h. tatsächlicher B2 Anteil ohne Berücksichtigung z.B. des Gewichts eines etwaigen Gegenions etc.) in einer Menge von etwa 0,3 mg bis etwa 7,7 mg, von etwa 0,4 mg bis etwa 7,4 mg, von etwa 0,4 mg bis etwa 7,1 mg, von etwa 0,5 mg bis etwa 6,9 mg, von etwa 0,6 mg bis etwa 6,6 mg, von etwa 0,6 mg bis etwa 6,3 mg, von etwa 0,7 mg bis etwa 6 mg, von etwa 0,8 mg bis etwa 5,7 mg, von etwa 0,8 mg bis etwa 5,6 mg, von etwa 1 mg bis etwa 5,5 mg, von etwa 1,1 mg bis etwa 5,3 mg, von etwa 1,3 mg bis etwa 5,2 mg, von etwa 1,4 mg bis etwa 5 mg, von etwa 1,5 mg bis etwa 4,9 mg, von etwa 1,7 mg bis etwa 4,8 mg, von etwa 1,8 mg bis etwa 4,6 mg, von etwa 2 mg bis etwa 4,5 mg, von etwa 2,1 mg bis etwa 4,3 mg, von etwa 2,2 mg bis etwa 4,2 mg, von etwa 2,4 mg bis etwa 4,1 mg, von etwa 2,5 mg bis etwa 3,9 mg, von etwa 2,7 mg bis etwa 3,8 mg, von etwa 2,8 mg bis etwa 3,6 mg, von etwa 2,9 mg bis etwa 3,5 mg und/oder von etwa 3,1 mg bis etwa 3,4 mg in den erfindungsgemäßen Zusammensetzungen vorliegen oder in jeglicher Kombinationen dieser Bereiche (z.B. von etwa 0,7 mg bis etwa 4,5 mg etc.). Besonders bevorzugt als Tagesdosis ist eine Menge von etwa 3,2 mg Vitamin B2 in den erfindungsgemäßen Zusammensetzungen.

Vorzugsweise liegt die Komponente Vitamin B12 in den erfindungsgemäßen Zusammensetzungen in einer (Tages-)Dosierung von etwa 20% bis etwa 440% der empfohlenen Tagesdosis, insbesondere von etwa 100% bis etwa 400% der empfohlenen Tagesdosis vor. Beispielsweise kann die Vitamin B12-Komponente (d.h. tatsächlicher Vitamin B12 Anteil ohne Berücksichtigung z.B. des Gewichts eines etwaigen Gegenions, Modifikation etc.) in einer Menge von etwa 0,5 µg bis etwa 11 µg, von etwa 0,6 µg bis etwa 10,5 µg, von etwa 0,6 µg bis etwa 10 µg, von etwa 0,7 µg bis etwa 9,5 µg, von etwa 0,8 µg bis etwa 9 µg, von etwa 0,8 µg bis etwa 8,5 µg, von etwa 0,9 µg bis etwa 8 µg, von etwa 0,9 µg bis etwa 7,5 µg, von etwa 1 µg bis etwa 7,3 µg, von etwa 1,1 µg bis etwa 7 µg, von etwa 1,1 µg bis etwa 6,8 µg, von etwa 1,3 µg bis etwa 6,5 µg, von etwa 1,4 µg bis etwa 6,3 µg, von etwa 1,5 µg bis etwa 6 µg, von etwa 1,6 µg bis etwa 5,8 µg, von etwa 1,8 µg bis etwa 5,5 µg, von etwa 1,9 µg bis etwa 5,3 µg, von etwa 2 µg bis etwa 5 µg, von etwa 2,1 µg bis etwa 4,8 µg, von etwa 2,3 µg bis etwa 4,5 µg, von etwa 2,4 µg bis etwa 4,2 µg, von etwa 2,5 µg bis etwa 4 µg, von etwa 2,6 µg bis etwa 3,7 µg, von etwa 2,8 µg bis etwa 3,5 µg und/oder von etwa 2,9 µg bis etwa 3,2 µg in den erfindungsgemäßen Zusammensetzungen vorliegen oder in jeglicher Kombinationen dieser Bereiche (z.B. von etwa 0,9 µg bis etwa 5,3 µg etc.). Besonders bevorzugt als Tagesdosis ist eine Menge von etwa 3 µg Vitamin B12 in den erfindungsgemäßen Zusammensetzungen.

Vorzugsweise liegt die Komponente Folsäure in den erfindungsgemäßen Zusammensetzungen in einer (Tages-)Dosierung von etwa 30% bis etwa 600% der empfohlenen Tagesdosis, insbesondere von etwa 50% bis etwa 400% der empfohlenen Tagesdosis vor. Beispielsweise kann die Folsäure-Komponente (d.h. tatsächlicher Folsäure Anteil ohne Berücksichtigung z.B. des Gewichts eines etwaigen Gegenions, Modifikation etc.) in einer Menge von etwa 30 µg bis etwa 600 µg, von etwa 50 µg bis etwa 590 µg, von etwa 70 µg bis etwa 580 µg, von etwa 90 µg bis etwa 570 µg, von etwa 110 µg bis etwa 560 µg, von etwa 130 µg bis etwa 550 µg, von etwa 150 µg bis etwa 540 µg, von etwa 170 µg bis etwa 530 µg, von etwa 190 µg bis etwa 520 µg, von etwa 210 µg bis etwa 510 µg, von etwa 230 µg bis etwa 500 µg, von etwa 250 µg bis etwa 490 µg, von etwa 270 µg bis etwa 480 µg, von etwa 290 µg bis etwa 470 µg, von etwa 310 µg bis etwa 460 µg, von etwa 330 µg bis etwa 450 µg, von etwa 350 µg bis etwa 440 µg, von etwa 360 µg bis etwa 430 µg, von etwa 370 µg bis etwa 420 µg, von etwa 380 µg bis etwa 410 µg und/oder von etwa 390 µg bis etwa 400 µg in den erfindungsgemäßen Zusammensetzungen vorliegen oder in jeglicher Kombinationen dieser Bereiche (z.B. von etwa 150 µg bis etwa 440 µg etc.). Besonders bevorzugt als Tagesdosis ist eine Menge von etwa 400 µg Folsäure in den erfindungsgemäßen Zusammensetzungen. Vorzugsweise liegt die Komponente Vitamin C in den erfindungsgemäßen Zusammensetzungen in einer (Tages-)Dosierung von etwa 25% bis etwa 500% der empfohlenen Tagesdosis, insbesondere von etwa 62% bis etwa 400% der empfohlenen Tagesdosis vor. Beispielsweise kann die Vitamin C-Komponente (d.h. tatsächlicher Vitamin C Anteil ohne Berücksichtigung z.B. des Gewichts eines etwaigen Gegenions, Modifikation etc.) in einer Menge von etwa 12,5 mg bis etwa 250 mg, von etwa 15 mg bis etwa 245 mg, von etwa 17,5 mg bis etwa 240 mg, von etwa 20 mg bis etwa 235 mg, von etwa 22,5 mg bis etwa 230 mg, von etwa 25 mg bis etwa 225 mg, von etwa 27,5 mg bis etwa 220 mg, von etwa 30 mg bis etwa 215 mg, von etwa 35 mg bis etwa 210 mg, von etwa 40 mg bis etwa 205 mg, von etwa 45 mg bis etwa 200 mg, von etwa 50 mg bis etwa 195 mg, von etwa 55 mg bis etwa 190 mg, von etwa 60 mg bis etwa 185 mg, von etwa 65 mg bis etwa 180 mg, von etwa 70 mg bis etwa 175 mg, von etwa 75 mg bis etwa 170 mg, von etwa 80 mg bis etwa 165 mg, von etwa 85 mg bis etwa 160 mg, von etwa 90 mg bis etwa 155 mg, von etwa 95 mg bis etwa 150 mg, von etwa 100 mg bis etwa 145 mg, von etwa 105 mg bis etwa 140 mg, von etwa 110 mg bis etwa 135 mg und/oder von etwa 115 mg bis etwa 130 mg in den erfindungsgemäßen Zusammensetzungen vorliegen oder in jeglicher Kombinationen dieser Bereiche (z.B. von etwa 27,5 mg bis etwa 170 mg etc.). Besonders bevorzugt als Tagesdosis ist eine Menge von etwa 120 mg Vitamin C in den erfindungsgemäßen Zusammensetzungen.

Vorzugsweise liegt die Komponente Zink in den erfindungsgemäßen Zusammensetzungen in einer (Tages-)Dosierung von etwa 15% bis etwa 350% der empfohlenen Tagesdosis, insbesondere von etwa 50% bis etwa 300% der empfohlenen Tagesdosis vor. Beispielsweise kann die Zink-Komponente (d.h. tatsächlicher Zink Anteil ohne Berücksichtigung z.B. des Gewichts eines etwaigen Gegenions, Modifikation etc.) in einer Menge von etwa 0,8 mg bis etwa 17,5 mg, von etwa 0,9 mg bis etwa 17 mg, von etwa 1 mg bis etwa 16,5 mg, von etwa 1,1 mg bis etwa 16 mg, von etwa 1,3 mg bis etwa 15,5 mg, von etwa 1,4 mg bis etwa 15 mg, von etwa 1,5 mg bis etwa 14,5 mg, von etwa 1,6 mg bis etwa 14 mg, von etwa 1,8 mg bis etwa 13,5 mg, von etwa 1,9 mg bis etwa 13 mg, von etwa 2 mg bis etwa 12,5 mg, von etwa 2,1 mg bis etwa 12 mg, von etwa 2,3 mg bis etwa 11,5 mg, von etwa 2,4 mg bis etwa 11 mg, von etwa 2,5 mg bis etwa 10,5 mg, von etwa 2,6 mg bis etwa 10 mg, von etwa 2,8 mg bis etwa 9,5 mg, von etwa 2,9 mg bis etwa 9 mg, von etwa 3 mg bis etwa 8,5 mg, von etwa 3,3 mg bis etwa 8 mg, von etwa 3,5 mg bis etwa 7,5 mg, von etwa 3,8 mg bis etwa 7 mg, von etwa 4 mg bis etwa 6,5 mg, von etwa 4,3 mg bis etwa 6 mg und/oder von etwa 4,5 mg bis etwa 5,5 mg in den erfindungsgemäßen Zusammensetzungen vorliegen oder in jeglicher Kombinationen dieser Bereiche (z.B. von etwa 1,5 mg bis etwa 9,5 mg etc.). Besonders bevorzugt als Tagesdosis ist eine Menge von etwa 5 mg Zink in den erfindungsgemäßen Zusammensetzungen.

Vorzugsweise liegt die Komponente Magnesium in den erfindungsgemäßen Zusammensetzungen in einer (Tages-)Dosierung von etwa 5% bis etwa 150% der empfohlenen Tagesdosis, insbesondere von etwa 15% bis etwa 100% der empfohlenen Tagesdosis vor. Beispielsweise kann die Magnesium-Komponente (d.h. tatsächlicher Magnesium Anteil ohne Berücksichtigung z.B. des Gewichts eines etwaigen Gegenions, Modifikation etc.) in einer Menge von etwa 19 mg bis etwa 570 mg, von etwa 22,8 mg bis etwa 532 mg, von etwa 26,6 mg bis etwa 513 mg, von etwa 30,4 mg bis etwa 494 mg, von etwa 34,2 mg bis etwa 475 mg, von etwa 38 mg bis etwa 456 mg, von etwa 41,8 mg bis etwa 437 mg, von etwa 45,6 mg bis etwa 418 mg, von etwa 49,4 mg bis etwa 399 mg, von etwa 53,2 mg bis etwa 380 mg, von etwa 57 mg bis etwa 361 mg, von etwa 60,8 mg bis etwa 342 mg, von etwa 64,6 mg bis etwa 323 mg, von etwa 68,4 mg bis etwa 304 mg, von etwa 72,2 mg bis etwa 285 mg, von etwa 76 mg bis etwa 266 mg, von etwa 79,8 mg bis etwa 247 mg, von etwa 82,7 mg bis etwa 228 mg, von etwa 85,5 mg bis etwa 209 mg, von etwa 88,4 mg bis etwa 190 mg, von etwa 90,3 mg bis etwa 171 mg, von etwa 92,2 mg bis etwa 152 mg, von etwa 94,1 mg bis etwa 139,3 mg, von etwa 96 mg bis etwa 126,7 mg und/oder von etwa 97,9 mg bis etwa 114 mg in den erfindungsgemäßen Zusammensetzungen vorliegen oder in jeglicher Kombinationen dieser Bereiche (z.B. von etwa 41,8 mg bis etwa 247 mg etc.). Besonders bevorzugt als Tagesdosis ist eine Menge von etwa 100 mg Magnesium in den erfindungsgemäßen Zusammensetzungen.

Vorzugsweise liegt die Komponente Eisen in den erfindungsgemäßen Zusammensetzungen in einer (Tages-)Dosierung von etwa 5% bis etwa 300% der empfohlenen Tagesdosis, insbesondere von etwa 15% bis etwa 200% der empfohlenen Tagesdosis vor. Beispielsweise kann die Eisen-Komponente (d.h. tatsächlicher Eisen Anteil ohne Berücksichtigung z.B. des Gewichts eines etwaigen Gegenions, Modifikation etc.) in einer Menge von etwa 0,7 mg bis etwa 42 mg, von etwa 1,1 mg bis etwa 40,6 mg, von etwa 1,5 mg bis etwa 39,2 mg, von etwa 2 mg bis etwa 37,8 mg, von etwa 2,4 mg bis etwa 36,4 mg, von etwa 2,8 mg bis etwa 35 mg, von etwa 3,2 mg bis etwa 33,6 mg, von etwa 3,6 mg bis etwa 32,2 mg, von etwa 4,1 mg bis etwa 30,8 mg, von etwa 4,5 mg bis etwa 29,4 mg, von etwa 4,9 mg bis etwa 28 mg, von etwa 5,3 mg bis etwa 26,6 mg, von etwa 5,7 mg bis etwa 25,2 mg, von etwa 6,2 mg bis etwa 23,8 mg, von etwa 6,6 mg bis etwa 22,4 mg, von etwa 7 mg bis etwa 21 mg, von etwa 7,7 mg bis etwa 20,3 mg, von etwa 8,4 mg bis etwa 19,6 mg, von etwa 9,1 mg bis etwa 18,9 mg, von etwa 9,8 mg bis etwa 18,2 mg, von etwa 10,5 mg bis etwa 17,5 mg, von etwa 11,2 mg bis etwa 16,8 mg, von etwa 11,9 mg bis etwa 16,1 mg, von etwa 12,6 mg bis etwa 15,4 mg und/oder von etwa 13,3 mg bis etwa 14,7 mg in den erfindungsgemäßen Zusammensetzungen vorliegen oder in jeglicher Kombinationen dieser Bereiche (z.B. von etwa 3,2 mg bis etwa 20,3 mg etc.). Besonders bevorzugt als Tagesdosis ist eine Menge von etwa 14 mg Eisen in den erfindungsgemäßen Zusammensetzungen.

Vorzugsweise liegt die Komponente (Bio)-Flavonoide in den erfindungsgemäßen Zusammensetzungen in einer Dosierung von etwa 20 mg bis etwa 200 mg vor.

Vorzugsweise liegt Schwarzer Pfeffer-Extrakt mit Piperin in den erfindungsgemäßen Zusammensetzungen in einer Dosierung von etwa 1 mg bis etwa 7,5 mg vor.

Die erfindungsgemäßen Zusammensetzungen können gemäß im Stand der Technik allgemein bekannter Verfahren in verschiedenen Darreichungsformen hergestellt und konfektioniert werden. Vorzugsweise werden die erfindungsgemäßen Zusammensetzungen in Form von Kapseln, Dragees, Tabletten, Granulat und/oder als Flüssiganwendung konfektioniert. Kapseln, insbesondere Pulverkapseln mit Gelatine- und oder Cellulosehülle sind besonders bevorzugt. Kapseln, Dragees und/oder Tabletten enthalten ferner vorzugsweise eine Tagesdosis der erfindungsgemäßen Zusammensetzungen.

In einem weiteren Aspekt betrifft die vorliegende Erfindung die Verwendung der efindungsgemäßen Zusammensetzungen als Nahrungsergänzungsmittel. Insbesondere betrifft die vorliegende Erfindung in diesem Zusammenhang die Verwendung der erfindungsgemäßen Zusammensetzungen zur Nahrungsergänzung bei Personen, die weibliche Hormonpräparate zu sich nehmen, z.B. hormonelle Kontrazeptiva, und/oder ggf. zur Nahrungsergänzung nach Absetzen der Einnahme dieser Hormonpräparate (z.B. der hormonellen Kontrazeptiva). Die Hormonpräparate, insbesondere die hormonellen Kontrazeptiva, können beispielsweise ausgewählt werden aus der Gruppe bestehend aus oralen und parenteralen Kontrazeptiva. Mögliche Beispiele für orale Kontrazeptiva sind die Antibabypille (z.B.: mit Östrogenen und Gestagenen) sowie die Minipille (z.B. mit Gestagenen). Mögliche Beispiele für parenterale Kontrazeptiva sind die Dreimonatsspritze, Vaginalringe, Hormonpflaster, Hormonimplantate und die Hormonspirale.

Der Zeitraum nach Absetzen der Einnahme von hormonellen Kontrazeptiva betrifft insbesondere den Zeitraum bis zum Eintritt einer Schwangerschaft und/oder die frühen Phasen der Schwangerschaft.

Ferner betrifft die vorliegende Erfindung die Verwendung der erfindungsgemäßen Zusammensetzungen zur Behandlung und/oder Prävention von Ernährungsmangelzuständen, insbesondere zur Behandlung und/oder Prävention von Ernährungsmangelzuständen die auf die Gabe hormoneller Kontrazeptiva zurückzuführen sind, z.B. bei Vitamin B2-Mangel, Vitamin B6-Mangel, Vitamin B12-Mangel, Folsäure-Mangel, Vitamin C-Mangel, Zink-Mangel und/oder Magnesium-Mangel (unter der Voraussetzung, dass der entsprechende Mangelstoff in der Zusammensetzung enthalten ist).

Die nachfolgenden Beispiele dienen der näheren Erläuterung des Erfindungsgegenstandes, ohne ihn auf diese beschränken zu wollen.

### Beispiel 1

Eine besonders bevorzugte Zusammensetzung der vorliegenden Erfindung umfasst folgende Inhaltsstoffe:

| **Komponente** | **Menge (Tagesdosis)** | **% der empfohlenen Tagesdosis** |
|---|---|---|
| Pyridoxinhydrochlorid (Vitamin B6) | 7,3 mg | 300 |
| Riboflavin (Vitamin B2) | 3,2 mg | 200 |
| 2% Hydroxycobalamin (Vitamin B12) | 0,15 mg | 300 |
| Folsäure | 400 µg | 200 |
| Calciumascorbat | 147 mg | 200 |
| Zinkglukonat | 42,4 mg | 33 |
| Magnesium | 100 mg | 33 |

Jeweils eine Tagesdosis dieser Zusammensetzung wurde in Gelatinekapseln abgefüllt, stellt also eine Formulierung für die Einnahme einmal am Tag ("once per day") dar. Die Zusammensetzung lässt sich selbstverständlich auch für eine Einnahme zweimal am Tag ("twice per day") oder dreimal am Tag, zum Beispiel zu den Mahlzeiten, formulieren, in dem 50% bzw. 33% der oben angegebenen Mengen verwendet werden.

### Beispiel 2

Eine besonders bevorzugte Zusammensetzung der vorliegenden Erfindung umfasst folgende Inhaltsstoffe:

| **Komponente** | **Menge (Tagesdosis)** | **% der empfohlenen Tagesdosis** |
|---|---|---|
| Pyridoxinhydrochlorid (Vitamin B6) | 7,3 mg | 300 |
| Riboflavin (Vitamin B2) | 3,2 mg | 200 |
| Methylcobalamin (Vitamin B12) | 3 µg | 300 |
| Folsäure | 400 µg | 200 |
| Calciumascorbat | 147 mg | 200 |
| Zinkglukonat | 42,4 mg | 33 |
| Magnesium | 100 mg | 33 |

Jeweils eine Tagesdosis dieser Zusammensetzung wurde in Gelatinekapseln abgefüllt. Die Zusammensetzung lässt sich selbstverständlich auch für eine Einnahme zweimal am Tag ("twice per day") oder dreimal am Tag, zum Beispiel zu den Mahlzeiten, formulieren, in dem 50% bzw. 33% der oben angegebenen Mengen verwendet werden.

### Beispiel 3

Die Zusammensetzungen aus Beispiel 1 und Beispiel 2 wurden zu einer Tablette enthaltend jeweils eine Tagesdosis (unter Zuhilfenahme von Lactose, Magnesiumstearat und Glycerin) zu einfachen Tabletten verpresst.

## Patentansprüche

1. Zusammensetzung, umfassend
i) Vitamin B6 oder Salze davon,
ii) Vitamin B2 und/oder Salze davon,
iii) Folsäure und/oder Salze davon,
iv) Vitamin B12 und/oder Salze davon,
v) Vitamin C und/oder Salze davon,
vi) Magnesium und/oder Salze davon, und
vii) Zink und/oder Salze davon,
wobei die Zusammensetzung vollständig frei von Vitamin A ist.

2. Zusammensetzung nach Anspruch 1, wobei die Zusammensetzung vollständig frei von ein, zwei, drei, vier, fünf, sechs oder sieben der Vitamine ausgewählt aus der Gruppe bestehend aus Vitamin B1, Vitamin B3, Vitamin B5, Vitamin B7, Vitamin D, Vitamin E, Vitamin K und deren Salzen, ist.

3. Zusammensetzung nach einem der vorhergehenden Ansprüche, wobei die Zusammensetzung mindestens eine weitere Substanz ausgewählt aus der Gruppe bestehend aus
i) Eisen und/oder Salze davon
ii) (Bio)Flavonoide, und
iii) Piperin umfasst.

4. Zusammensetzung nach Anspruch 3, wobei die Zusammensetzung mindestens zwei Substanzen ausgewählt aus der Gruppe bestehend aus i), ii) und iii) umfasst.

5. Zusammensetzung nach einem der vorhergehenden Ansprüche, wobei die Zusammensetzung Eisen, (Bio)Flavonoide und Piperin, oder jeweils Salze davon, umfasst.

6. Zusammensetzung nach einem der vorhergehenden Ansprüche, wobei die Zusammensetzung im Wesentlichen oder vollständig frei ist von:
i) pflanzlichen Extrakten,
ii) aus pflanzlichem Material isolierten Stoffen, und/oder
iii) weiblichen Hormonen.

7. Verwendung der Zusammensetzung nach einem der Ansprüche 1-6 als Nahrungsergänzungsmittel.

8. Verwendung nach Anspruch 7, wobei die Zusammensetzung zur Nahrungsergänzung bei Personen verwendet wird, die weibliche Hormonpräparate zu sich nehmen.

9. Verwendung nach Anspruch 7, wobei die Zusammensetzung zur Nahrungsergänzung bei Personen verwendet wird, die die Einnahme weiblicher Hormonpräparate abgesetzt haben.

10. Verwendung nach einem der Ansprüche 7 oder 9, wobei die weiblichen Hormonpräparate orale oder parenterale Kontrazeptiva sind.

11. Zusammensetzung nach einem der Ansprüche 1-6 zur Anwendung bei der Behandlung und/oder Prävention von Ernährungsmangelzuständen infolge der Einnahme von weiblichen Hormonpräparaten.

## Claims

1. Composition comprising
i) vitamin B6 or salts thereof,
ii) vitamin B2 and/or salts thereof,
iii) folic acid and/or salts thereof,
iv) vitamin B12 and/or salts thereof,
v) vitamin C and/or salts thereof,
vi) magnesium and/or salts thereof, and
vii) i) zinc and/or salts thereof,
wherein the composition is completely devoid of vitamin A.

2. Composition according to claim 1, wherein the composition is completely devoid of one, two, three, four, five, six or seven of the vitamins selected from the group consisting of vitamin B1, vitamin B3, vitamin B5, vitamin B7, vitamin D, vitamin E, vitamin K and their respective salts.

3. Composition according to any one of the preceding claims, wherein the composition comprises at least one further substance selected from the group consisting of
i) iron and/or salts thereof
ii) (bio)flavonoids, and
iii) piperine.

4. Composition according to claim 3, wherein the composition comprises at least two substances selected from the group consisting of i), ii) and iii).

5. Composition according to any one of the preceding claims, wherein the composition comprises iron, (bio)flavonoids and piperine, or their respective salts.

6. Composition according to any one of the preceding claims, wherein composition is essentially or completely devoid of:
i) herbal extracts
ii) compounds isolated from herbal material, and/or
iii) female hormones.

7. Use of a composition according to any one of claims 1 - 6 as dietary supplement.

8. Use according to claim 7, wherein the composition for dietary supplementation is used in individuals receiving female hormone preparations.

9. Use according to claim 7, wherein the composition for dietary supplementation is used in individuals which have discontinued intake of female hormone preparations.

10. Use according to one of claims 7 or 9, wherein the female hormone preparations are oral or parenteral contraceptives.

11. Composition according to any one of claims 1-6 for use in the treatment and/or prevention of nutritional deficiency conditions due to the intake of female hormone preparations.

## Revendications

1. Composition, comprenant
i) de la vitamine B6 ou des sels de celle-ci,
ii) de la vitamine B2 et/ou des sels de celle-ci,
iii) de l'acide folique et/ou des sels de celui-ci,
iv) de la vitamine B12 et/ou des sels de celle-ci,
v) de la vitamine C et/ou des sels de celle-ci,
vi) du magnésium et/ou des sels de celui-ci, et
vii) du zinc et/ou des sels de celui-ci,
la composition est totalement dépourvu de vitamine A.

2. Composition selon la revendication 1, la composition est totalement dépourvu de une, deux, trois, quatre, cinq, six ou sept des vitamines choisies dans le groupe comprenant la vitamine B1, la vitamine B3, la vitamine B5, la vitamine B7, la vitamine D, la vitamine E, la vitamine K et leurs sels.

3. Composition selon l'une des revendications précédentes, la composition comprenant au moins une autre substance choisie dans le groupe comprenant
i) du fer et/ou des sels de celui-ci
ii) des (bio)flavonoïdes, et
iii) de la pipérine.

4. Composition selon la revendication 3, la composition comprenant au moins deux substances choisies dans le groupe comprenant les composants i), ii) et iii).

5. Composition selon l'une des revendications précédentes, la composition comprenant du fer, des (bio)flavonoïdes et de la pipérine, ou des sels respectifs de ceux-ci.

6. Composition selon l'une des revendications précédentes, la composition étant substantiellement ou complètement dépourvue :
i) d'extraits végétaux,
ii) de substances isolées de matériau végétal, et/ou
iii) d'hormones féminines.

7. Utilisation de la composition selon l'une des revendications 1 à 6, comme complément alimentaire.

8. Utilisation selon la revendication 7, la composition de complément alimentaire étant utilisée chez des personnes qui prennent des préparations d'hormones féminines.

9. Utilisation selon la revendication 7, la composition de complément alimentaire étant utilisée chez des personnes qui ont arrêté de prendre des préparations d'hormones féminines.

10. Utilisation selon l'une des revendications 7 ou 9, les préparations d'hormones féminines étant des contraceptifs oraux ou parentéraux.

11. Composition selon l'une des revendications 1 à 6, pour son utilisation dans le traitement et/ou la prévention des états de carence alimentaire à la suite de la prise de préparations d'hormones féminines.
